(19) Europäisches Patentamt
European Patent Office
Office européen des brevets

(11) **EP 3 373 173 A1**

(12) **EUROPEAN PATENT APPLICATION**

(43) Date of publication:
**12.09.2018 Bulletin 2018/37**

(51) Int Cl.:
**G06F 19/00** (2018.01)   **G01N 33/48** (2006.01)
**A61B 5/08** (2006.01)   **G01N 33/497** (2006.01)

(21) Application number: **17160286.5**

(22) Date of filing: **10.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **Ron Martinus Laurentius, VAN LIESHOUT**
**5656 AE Eindhoven (NL)**

• **Ronaldus Maria, AARTS**
**5656 AE Eindhoven (NL)**
• **Rick, BEZEMER**
**5656 AE Eindhoven (NL)**
• **Murtaza, BULUT**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(54) **METHOD AND APPARATUS FOR MONITORING A SUBJECT**

(57)    According to an aspect, there is provided a method of monitoring a subject, the method comprising obtaining measurements of a parameter relating to one or more volatile organic compounds, VOCs, in one or more outputs of the subject over time, wherein the one or more VOCs are associated with the food or produced while the food is metabolised or digested by the subject; and processing the measurements to determine an amount and/or type of food ingested by the subject and/or a health status of the subject.

Figure 1

**EP 3 373 173 A1**

**Description**

Technical Field of the Invention

The invention relates to a method and apparatus for monitoring a subject, and in particular to a method and apparatus for monitoring an amount and/or type of food ingested by a subject and/or a health status of a subject.

Background to the Invention

Intake of food with the right nutrition levels is essential for living a healthy life. However, achieving a good balance of nutritional food can be especially challenging for elderly people, people with eating disorders like obesity or anorexia nervosa, and people suffering from degenerative diseases. Therefore systems have been provided that can track the food eaten by a person, for example using 'smart' cutlery and/or plates that measure the amount of food that has been removed from the plate or the number of times the cutlery has moved to the person's mouth. However, these solutions are relatively complicated and assume that the food that leaves the plate is eaten by the person, which is in some cases incorrect since a person could hide the food from (e.g. from a care provider) or throw it away. Moreover, although the total amount of food can be monitored, it can be difficult to distinguish how much of each different nutritional group has been consumed, such as vitamins, minerals, proteins, etc. Other systems can make use of one or more cameras to monitor the person.

Ideally the metabolic state of a person would be measured and related to the nutrition state. The metabolic system can be divided into approximately three pathways; carbohydrate, protein, and fat metabolism. These metabolic pathways have different intermediate and waste molecules, provide different degrees of metabolic energy (i.e. adenosine triphosphate (ATP), nicotinamide adenine dinucleotide (NADH)), require a different amount of oxygen ($O_2$), generate a different amount of carbon dioxide ($CO_2$), etc.

Deviations from the preferred metabolic pathways is often linked to disease or under- or mal-nutrition. There are several ways of measuring the metabolic state. In academic studies this is done using stable isotopes of carbon (13C), oxygen (18O), nitrogen (15N) and sulphur (34S). The digestive and metabolic system of a person will absorb these isotopes and use them in the metabolic processes. They can be processed into different molecules which can end up in the lungs (e.g. carbon dioxide ($CO_2$), volatile organic compounds (VOCs)), skin (VOC) and urine (waste products).

One of the best known metabolic markers for disease are ketones. Ketones are generated, for example, when the concentration of the hormone insulin, which regulates glucose intake into the cells, is too low, inhibiting the preferred glucose pathways in cellular respiration. This results in a shift towards protein metabolism which is associated with the formation of ketones. Ketones are VOCs which can be smelt (i.e. detected) on the person's breath. This is especially valuable for people with diabetes, in whom increased ketone levels indicate insufficient disease management.

As noted above, malnutrition due to insufficient food intake, hiding the food or disposing of the food can be missed by conventional monitoring systems. Current methods for detecting malnutrition rely heavily on monitoring the amount of food that is given to the person on a plate or brought to the mouth by 'smart' cutlery and does not account for the person disposing of or hiding food. Moreover, it is desired to track not only the intake of food but the absorption of the nutrition via the digestive system. In addition when specific supplements are given (i.e. minerals, vitamins, proteins, fat, etc.) it would be useful to trace the intake (and uptake) of these components by the body.

In chronically ill people that are receiving medication, it is important that the metabolic rate and the nutritional intake of the person is somewhat stable (or at least accounted for) to ensure proper uptake and therapeutic effect of the medication. When the person has a change in diet, gets an infection, has stress, or any other environmental change occurs, the balance between the medication dose and effect can be disturbed and this can lead to exacerbation of the person's condition. Therefore, it is important to detect the onset of any change in a person's nutritional intake and/or metabolic rate to allow early intervention to restore this balance, for example by optimising food intake, to prevent long-term imbalance and/or exacerbation.

State of the art systems for monitoring metabolism can measure breath by breath or mixing chamber oxygen consumption, carbon dioxide production, minute ventilation, anaerobic threshold detection, flow volume loops, lung subdivisions, and/or maximal voluntary ventilation. These measurements can be useful in determining aerobic capacity, VO2 (oxygen consumption) Peak, VO2 Maximum, work capacity, pulmonary function, and resting energy expenditure as well as many other clinical research outcomes. However, these systems require accurate measurement of the person via a mask, which makes it acceptable for point measurements but not for monitoring purposes.

The use of stable isotopes is mainly an academic tool and is difficult to integrate in day-to-day use in a low-cost system.

Therefore there is a need for an improved method and apparatus for monitoring an amount and/or type of food ingested by a subject and/or a health status of a subject.

Summary of the Invention

According to a first aspect, there is provided a method of monitoring a subject (e.g. an individual, a person, a patient, a user or otherwise a mammal), the method comprising obtaining measurements of a parameter relating to one or more volatile organic compounds, VOCs, in one or more outputs of the subject over time, wherein the one or more VOCs are associated with the food or produced while the food is metabolised or digested by the subject; and processing the measurements to determine an amount and/or type of food ingested by the subject and/or a health status of the subject. Therefore the invention provides a relatively simple, cost effective and relatively unobtrusive approach to monitoring a subject and providing information on their food intake and/or health status.

In some embodiments, the one or more outputs comprise one or more of breath, urine and sweat of the subject.

In some embodiments, the health status comprises one or more of a measure of a metabolic rate of the subject, a measure of the kidney function of the subject and a measure of the liver function of the subject. These health statuses can be determined since these functions contribute to the VOCs in the outputs of the subject.

In some embodiments, the step of obtaining comprises obtaining measurements of the parameter relating to the one or more VOCs in urine from the subject, and the step of processing comprises determining a measure of the kidney function of the subject from the measurements of the parameter relating to the one or more VOCs in the urine over time. The VOCs in urine is directly affected by the functioning of the kidney, so this provides a useful, yet unobtrusive way to measure kidney function. In these embodiments, the step of obtaining comprises obtaining measurements of the parameter relating to the one or more VOCs in at least one other output of the subject over time, and the step of processing comprises comparing the measurements of the parameter relating to the one or more VOCs in the urine over time to the measurements of the parameter relating to the one or more VOCs in the at least one other output to determine the measure of the kidney function of the subject. This embodiment has the advantage that it can show whether kidney function has changed through a comparison of the VOC parameter in urine to the VOC parameter in another output for which the kidney is not directly responsible. In effect, this embodiment uses the other output as a baseline for measuring the kidney function.

In some embodiments, the step of obtaining comprises obtaining measurements of the parameter relating to the one or more VOCs in a plurality of outputs of the subject over time, and the step of processing the measurements to determine the health status comprises comparing measurements of the parameter relating to the VOCs for each of the plurality of outputs. In these embodiments, the step of comparing comprises determining a ratio of the measurements of the parameter relating to the VOCs for a first output to the measurements of the parameter relating to the VOCs for a second output. These embodiments have the advantage that they can be used to show whether the subject has ingested a particular food (for example if the VOC(s) is/are present in each of the outputs). These embodiments also have the advantage that the VOCs in one output can be used as a baseline for the VOCs in the other output.

In some embodiments, the step of processing the measurements to determine the health status comprises comparing the measurements of the parameter relating to the one or more VOCs over time to previous measurements of the parameter relating to the one or more VOCs in the one or more outputs of the subject over time. This embodiment has the advantage that it can provide an indication of a change in the health status of the subject over time.

In some embodiments, the one or more VOCs comprises a first VOC that is produced when the food is metabolised or digested by the subject, and the step of processing comprises determining a measure of the metabolic rate of the subject and/or the amount and/or type of food ingested by the subject from the measurements of the parameter relating to the first VOC over time.

In some embodiments, the one or more VOCs comprises a first VOC that is produced when the food is metabolised or digested by the subject and a second VOC that is comprised in the food and is unchanged by metabolism or digestion in the subject, and the step of processing comprises determining a measure of the liver function of the subject from the measurements of the parameter relating to the first VOC over time relative to the measurements of the parameter relating to the second VOC over time. This embodiment has the advantage that it provides a simple way to measure the liver function of the subject.

In some embodiments, the one or more outputs are measured by one or more sensors.

In some embodiments, the one or more VOCs comprises any of S-Methyl thioacrylate, S-methyl3-(methylthio)thiopropionate), alliinase, sulfurous allicin, diallyl disulfide, allyl methyl sulfide, allyl methyl disulphide, allyl mercaptan, methanethiol, dimethyl sulphide, dimethylsulfoniopropionate, dimethyl disulphide, bis(methylthio)methane, dimethyl sulfoxide and dimethyl sulfone.

In some embodiments, the method further comprises the step of adding a tracer element to a food to be ingested by the subject, and the tracer element is a VOC or produces a VOC when the tracer element is metabolised or digested by the subject. This embodiment has the advantages that the sensors can be focused on a particular VOC (for example one that is not naturally found in a food to be ingested, or not naturally produced through digestion of the food), that a VOC can be produced in the case where a particular food does not normally result in a VOC in an output of the subject, and/or that it can be used to increase the amount of VOCs in the output(s) of the subject, and thus potentially increase the ease with which the VOCs can be measured.

In some embodiments, the measurements of the parameter relating to one or more VOCs in one or more outputs of the subject comprises measurements of the quantity of the one or more VOCs in the one or more outputs of the subject. In other embodiments, the parameter comprises the concentration (i.e. quantity per unit volume) of the one or more VOCs in the output(s) of the subject, the volume of the one or more VOCs in the output(s) of the subject, and/or the output rate of the one or more VOCs in the output(s) of the subject.

According to a second aspect, there is provided a computer program product comprising a computer readable medium having computer readable code embodied therein, the computer readable code being configured such that, on execution by a suitable computer or processor, the computer or processor is caused to perform any of the methods described above.

According to a third aspect, there is provided an apparatus for monitoring a subject, the apparatus comprising one or more sensors for measuring a parameter relating to one or more volatile organic compounds, VOCs, in one or more outputs of the subject over time, wherein the one or more VOCs are associated with the food or produced while the food is metabolised or digested by the subject; and a processing unit configured to process the measurements to determine an amount and/or type of food ingested by the subject and/or a health status of the subject. Therefore the invention provides a relatively simple, cost effective and relatively unobtrusive approach to monitoring a subject and providing information on their food intake and/or health status.

In some embodiments, the one or more outputs comprise one or more of breath, urine and sweat of the subject.

In some embodiments, the health status comprises one or more of a measure of a metabolic rate of the subject, a measure of the kidney function of the subject and a measure of the liver function of the subject. These health statuses can be determined since these functions contribute to the VOCs in the outputs of the subject.

In some embodiments, the one or more sensors are for measuring the parameter relating to the one or more VOCs in urine from the subject, and the processing unit is configured to process the measurements to determine a measure of the kidney function of the subject from the measurements of the parameter relating to the one or more VOCs in the urine over time. The VOCs in urine is directly affected by the functioning of the kidney, so this provides a useful, yet unobtrusive way to measure kidney function. In these embodiments, the one or more sensors are also for measuring the parameter relating to the one or more VOCs in at least one other output of the subject over time, and the processing unit is configured to compare the measurements of the parameter relating to the one or more VOCs in the urine over time to the measurements of the parameter relating to the one or more VOCs in the at least one other output to determine the measure of the kidney function of the subject. This embodiment has the advantage that it can show whether kidney function has changed through a comparison of the VOC parameter in urine to the VOC parameter in another output for which the kidney is not directly responsible. In effect, this embodiment uses the other output as a baseline for measuring the kidney function.

In some embodiments, the one or more sensors are for measuring the parameter relating to the one or more VOCs in a plurality of outputs of the subject over time, and the processing unit is configured to process the measurements to determine the health status by comparing measurements of the parameter relating to the VOCs for each of the plurality of outputs. In these embodiments, the processing unit is configured to compare the measurements by determining a ratio of the measurements of the parameter relating to the VOCs for a first output to the measurements of the parameter relating to the VOCs for a second output. These embodiments have the advantage that they can be used to show whether the subject has ingested a particular food (for example if the VOC(s) is/are present in each of the outputs). These embodiments also have the advantage that the VOCs in one output can be used as a baseline for the VOCs in the other output.

In some embodiments, the processing unit is configured to process the measurements to determine the health status by comparing the measurements of the parameter relating to the one or more VOCs over time to previous measurements of the parameter relating to the one or more VOCs in the one or more outputs of the subject over time. This embodiment has the advantage that it can provide an indication of a change in the health status over time.

In some embodiments, the one or more VOCs comprises a first VOC that is produced when the food is metabolised or digested by the subject, and the processing unit is configured to process the measurements by determining a measure of the metabolic rate of the subject and/or the amount and/or type of food ingested by the subject from the measurements of the parameter relating to the first VOC over time.

In some embodiments, the one or more VOCs comprises a first VOC that is produced when the food is metabolised or digested by the subject and a second VOC that is comprised in the food and is unchanged by metabolism or digestion in the subject, and the processing unit is configured to determine a measure of the liver function of the subject from the measurements of the parameter relating to the first VOC over time relative to the measurements of the parameter relating to the second VOC over time. This embodiment has the advantage that it provides a simple way to measure the liver function of the subject.

In some embodiments, the one or more VOCs comprises any of S-Methyl thioacrylate, S-methyl3-(methylthio)thiopropionate), alliinase, sulfurous allicin, diallyl disulfide, allyl methyl sulfide, allyl methyl disulphide, allyl mercaptan, methanethiol, dimethyl sulphide, dimethylsulfoniopropionate, dimethyl disulphide, bis(methylthio)methane, dimethyl sulfoxide and dimethyl sulfone.

In some embodiments, the measurements of the parameter relating to one or more VOCs in one or more outputs of the subject comprises measurements of the quantity of the one or more VOCs in the one or more outputs of the subject. In other embodiments, the parameter comprises the concentration (i.e. quantity per unit volume) of the one or more VOCs in the output(s) of the subject, the volume of the one or more VOCs in the output(s) of the subject, and/or the output rate of the one or more VOCs in the output(s) of the subject.

These and other aspects of the invention are apparent from and will be elucidated with reference to the embodiments described hereinafter.

It will be appreciated by those skilled in the art that two or more of the above-mentioned options, implementations, and/or aspects of the invention may be combined in any way deemed useful.

Brief Description of the Drawings

For a better understanding of the invention, and to show more clearly how it may be carried into effect, reference will now be made, by way of example only, to the accompanying drawings, in which:

Figure 1 is a schematic representation of the clearance of VOCs by the body;
Figure 2 is a block diagram of an apparatus according to an embodiment;
Figure 3 is a flow chart of a method according to an embodiment;
Figure 4 is a graph illustrating an exemplary model for the quantity of VOCs output by a subject over time; and
Figures 5, 6 and 7 illustrate respective exemplary processes that can be performed according to various embodiments.

Detailed Description of the Preferred Embodiments

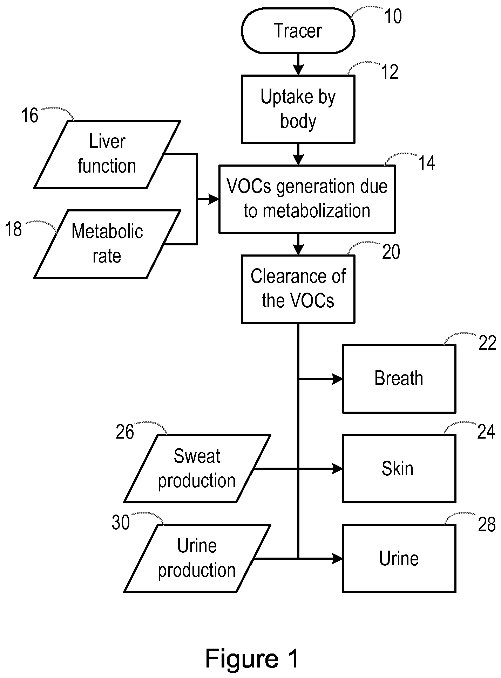

Figure 1 is a schematic representation of the clearance of volatile organic compounds (VOCs) by the body. Block 10 shows a tracer element, which can be present in food to be ingested by a subject (for example as part of an ingredient of the food), or specifically added to food to be ingested by a subject. A suitable tracer element could be asparagusic acid or garlic. Further details of possible tracer elements are provided below. The tracer is ingested or taken up by the subject, as shown by block 12. The subject's body generates VOCs due to metabolisation of the tracer (block 14), with the quantity of VOCs generated being dependent on the subject's liver function (block 16) and metabolic rate (block 18).

The body of the subject then starts to clear out the VOCs, i.e. get rid of them from the body (block 20). There are three main ways in which the body can excrete VOCs, they are in the breath (block 22), through the skin, via sweat (block 24), with the quantity of VOCs depending on the rate of sweat production (block 26), and in urine (block 28), with the quantity of VOCs depending on the rate of urine production (block 30).

As noted above, it is important to be able to monitor the amount of food that has been ingested (i.e. eaten) by a subject, the type of food ingested by a subject and/or the health status of a subject. Since the quantity of VOCs in an output of the subject depends on the nature and quantity of the food ingested by the subject, as well as the functioning of the body of the subject (e.g. metabolism, liver function, etc.), monitoring the quantity of VOCs in an output of the subject can provide an indication of the amount and/or type of food that has been ingested and/or a health status of the subject.

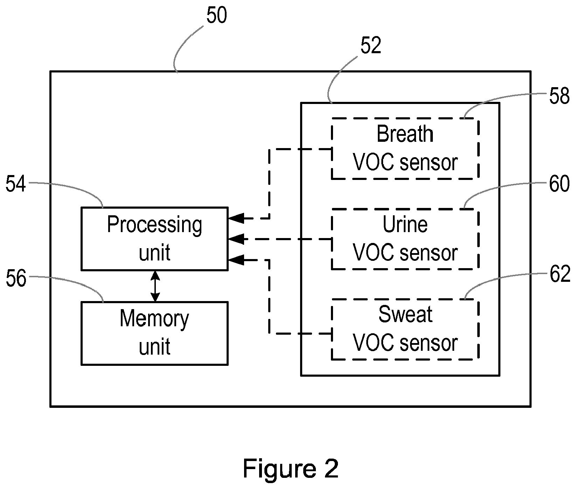

Figure 2 shows an apparatus 50 for monitoring a subject according to an embodiment. The apparatus 50 comprises one or more VOC sensors 52 that are measuring a parameter relating to one or more VOCs in an output from the subject and a processing unit 54 that receives the measurements of the VOC parameter from the one or more sensors 52 and processes the measurements to determine an amount and/or type of food that has been ingested and/or a health status of the subject. The one or more VOC sensors 52 preferably measure the VOCs that are associated with food that is ingested by the subject, and/or that are produced by food as it is metabolised or digested by the subject.

In preferred embodiments, the parameter relating to one or more VOCs is the quantity of the one or more VOCs in the output from the subject. In other embodiments, the parameter relating to one or more VOCs is the concentration of the one or more VOCs in the output from the subject (i.e. the quantity per unit volume), a volume of the one or more VOCs in the output from the subject, and/or an output rate of one or more VOCs in the output of the subject. It will be appreciated that these VOC parameters are related and a measurement of one of the VOC parameters can be used to infer or derive another of the VOC parameters. For example a measurement of the concentration of the one or more VOCs can be derived from a measurement of the quantity of the one or more VOCs and information on the volume of the output over which that quantity was measured, and vice versa. Thus, in preferred embodiments, the one or more VOC sensors 52 measure the quantity of one or more VOCs in the output from the subject. However, in other embodiments, the one or more VOC sensors 52 can measure the concentration of one or more VOCs in the output. In either case, the volume of the output can also be measured (for example by a separate volume sensor, or by the VOC sensor(s) 52. In some embodiments, several different parameters can be measured and used to determine the food ingested and/or health status of the subject.

In the following description of the invention, the invention will mainly be described with reference to measurements of the *quantity* of the VOCs, but it will be appreciated that the invention and techniques described herein can equally be applied to other parameters relating to VOCs, such as concentration or volume of VOCs.

In some embodiments, the apparatus 50 is in a form that can be worn or carried by the subject (e.g. the apparatus 50 is portable). In alternative embodiments, part of the apparatus 50, specifically one or more of the sensors 52, are in a form that can be worn or carried by the subject, and the other part of the apparatus 50, e.g. the part comprising the processing unit 54, is not carried or worn by the subject, for example it is in or part of an electronic device such as a laptop, desktop computer or server that can be connected to the one or more sensors 52 to receive the measurements of the quantity of VOCs in an output of the subject. In some embodiments, the one or more sensors 52 can be integrated into a pair of glasses, with the sensor(s) 52 being located near to the mouth or nose to measure VOCs in the breath of the subject and/or VOCs in the sweat of the subject.

The processing unit 54 can receive the measurements of VOC quantity in real-time or near real-time, and the processing unit 54 can process the measurements in real-time or near real-time, or alternatively the processing unit 54 can store the measurements for processing at a later stage (for example at the end of each hour, day, or other monitoring period), for example in memory unit 56. In addition, the processing unit 54 can control the operation of the apparatus 50, for example activating and/or deactivating the one or more sensors 52 as required.

The processing unit 54 can be implemented in numerous ways, with software and/or hardware, to perform the various functions described below. The processing unit 54 may comprise one or more microprocessors or digital signal processor (DSPs) that may be programmed using software or computer program code to perform the required functions and/or to control components of the processing unit 54 to effect the required functions. The processing unit 54 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In various implementations, the processing unit 54 may be associated with or comprise one or more memory units 56 such as volatile and non-volatile computer memory such as RAM, PROM, EPROM, and EEPROM. The processing unit 54 or associated memory unit 56 can also be used for storing program code that can be executed by a processor in the processing unit 54 to perform the method described herein. The memory unit 56 can also be used to store signals or measurements received from the one or more VOC sensors 52. This is particularly useful where the measurements from the one or more sensors 52 are stored and processed at a later stage.

The one or more sensors 52 can comprise a breath VOC sensor 58 that is for measuring the quantity of VOCs in the breath of the subject. In some embodiments, the breath VOC sensor 58 can be part of a mask that can be worn by the subject, it can be part of a nasal tube, or it can otherwise be configured to be located in the exhaled breath flow from the subject.

The one or more sensors 52 can also or alternatively comprise a urine VOC sensor 60 that is for measuring the quantity of VOCs in the urine of the subject. In some embodiments, the urine VOC sensor 60 can be integrated or part of a toilet that is frequently used by the subject (e.g. a toilet in the home of the subject).

The one or more sensors 52 can also or alternatively comprise a sweat VOC sensor 62 that is for measuring the quantity of VOCs in the sweat of a the subject. The sweat VOC sensor 62 can be integrated into an item of clothing of the subject, or it can be part of another type of wearable item, such as an armband or wrist watch.

Various types of sensors that can measure VOCs are known to those skilled in the art, and thus are only described briefly herein. For example, so-called e-nose devices are known that can measure VOCs, and that can be integrated in a wearable device. Also sensors can be made for sensing specific VOCs using molecular imprint technology. Another suitable sensor is a gas chromatograph. In some embodiments, the VOC sensor can be a weight-detectable sensor modified with porous metal-organic framework (MOF) layers. The large surface area and regulated nanospace of the MOF layers enhances their sensitivity and selectivity for VOC sensing. VOCs can be detected by measuring the frequency changes in the sensors.

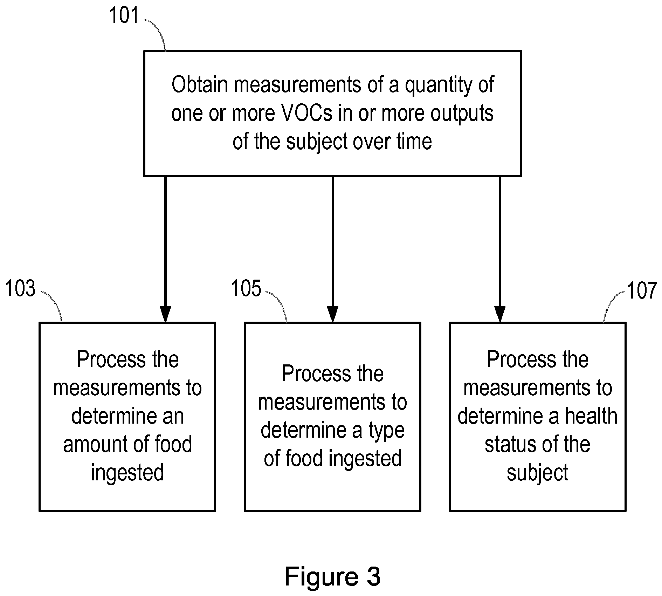

The flow chart in Figure 3 shows a method of monitoring a subject according to an embodiment. The method can be performed by apparatus 50, and in particular by the processing unit 54.

In a first step, step 101, the processing unit 54 obtains measurements of a parameter relating to one or more VOCs (for example a quantity of one or more VOCs) in one or more outputs of the subject over time. Step 101 can comprise the processing unit 54 obtaining (e.g. retrieving) measurements from a memory unit 56, or receiving the measurements directly from the one or more sensors 52. In some embodiments, step 101 can comprise controlling the one or more sensors 52 to start making measurements of the parameter relating to one or more VOCs (for example the quantity of VOCs). The measurements preferably relate to one or more VOCs that are associated with food that is ingested by the subject, or produced by food as it is metabolised or digested by the subject.

In some embodiments, the one or more outputs of the subject include breath, urine and/or sweat, and thus the one or more sensors 52 are arranged or configured to measure the quantity of VOCs in breath, urine and/or sweat.

Next, the measurements of the parameter relating to one or more VOCs (for example quantity of the one or more VOCs) over time are processed to determine information about the subject. In particular, in step 103, the measurements are processed to determine an amount of food ingested by the subject. In addition or alternatively, the measurements can be processed to determine a type of food ingested by the subject (step 105). In addition or alternatively, the measurements can be processed to determine a health status of the subject (step 107).

As noted above, the VOCs measured by the sensor(s) 52 are VOCs that are produced by the body of the subject from the food that is ingested by the subject. In some embodiments, the food to be ingested can be modified or 'spiked' with an ingredient or chemical (referred to as a tracer element) that will produce VOCs when the food is metabolised or digested by the body of the subject. Thus, in some embodiments, the method additionally comprises the step of adding a tracer element to a food to be ingested by the subject. The tracer element can be a VOC, or an element that is metabolised or digested to produce a VOC. Some examples are provided below.

The tracer element can be a natural tracer, which after metabolisation is modified such that it is secreted from the body, e.g. in breath, sweat or urine. In this embodiment the natural tracer may be an existing ingredient or component of a food to be ingested (i.e. it is not necessary to add the tracer element to the food). However, it will be appreciated that such a natural tracer can be deliberately added to a food. Examples of natural tracers include:

- Asparagusic acid, which breaks down into sulfuric compounds (S-Methyl thioacrylate and S-methyl3-(methylthio)thiopropionate) which can be detected in the urine of a subject and in other outputs;
- Garlic. Digesting garlic releases an enzyme called alliinase, which converts odourless alliin molecules into pungent, sulfurous allicin. Allicin is the major contributor to the aroma of freshly-chopped garlic. It is unstable and quickly breaks down into several other sulfur-containing compounds, including diallyl disulfide, allyl methyl sulfide, allyl methyl disulfide and allyl mercaptan. The body metabolises most of these compounds within a few hours, but allyl methyl sulfide is slower to break down and stays in the body longer than the others, sometimes up to a day or two. These components circulate in the blood stream and are secreted via the lungs (e.g. in breath), via the skin (e.g. in sweat), and via urine;
- Methanethiol, which occurs naturally in certain foods, such as some nuts and cheese;
- Dimethyl sulphide, which is a breakdown product of dimethylsulfoniopropionate (DMSP);
- Dimethyl disulfide (DMDS), which is used as a food additive in onion, garlic, cheese, meats, soups, savoury dishes, and fruit dishes;
- Bis(methylthio)methane, which is a component of truffle flavour;
- Dimethyl sulfoxide, which is a chemical solvent also used in medication;
- Dimethyl sulfone, which occurs naturally in some primitive plants, is present in small amounts in many foods and beverages, and is marketed as a dietary supplement.

In alternative embodiments, the tracer element can be a synthetic tracer which after metabolisation is modified such that it is secreted from the body, e.g. in breath, sweat or urine. In this embodiment the synthetic tracer may not be an existing ingredient or component of a food to be ingested, but it can be added to a food prior to ingestion. Examples of synthetic tracers include components such as allyl methyl sulfide or allyl mercaptan.

Steps 103, 105 and 107 can comprise processing the measurements to determine a change in the parameter relating to the VOCs in the output of the subject over time. For example steps 103, 105 and 107 can comprise processing the measurements to determine a change in quantity of the VOCs in the output of the subject over time. As shown in Figure 1, the change in quantity in VOCs depends on the quantity of VOCs in the food (or the quantity of ingredients that are processed by the body to produce VOCs), the liver function of the subject, the metabolic rate of the subject, the rate of sweat production and the rate of urine production (which is related to kidney function). Thus, step 103 can comprise determining an amount of food ingested based on a change in quantity in the VOCs, and in particular based on an amount of increase in VOCs after ingesting food. Step 105 can comprise determining that a particular type of food has been ingested based on the presence of certain VOCs in the output(s). Step 107 can comprise determining a health status of the subject (e.g. liver function, metabolism, kidney function, etc.) based on changes in quantity of the VOCs, such as rates of increase and/or decrease in the measured quantity. In a specific embodiment, since the breakdown of precursors of a VOC (e.g. garlic) into VOCs is performed by the liver, the rate of change of the VOCs over time will provide an indication of the liver function.

In a particular embodiment of step 107, the measurements obtained in step 101 can be measurements of the quantity of the one or more VOCs in urine from the subject, step 107 can and the measurements of VOCs in urine can be processed to determine a measure of the kidney function. In some embodiments, step 101 can comprise obtaining measurements of the quantity of the one or more VOCs in at least one other output (in addition to urine), and step 107 can comprise comparing the urine VOC measurements to the measurements of the quantity of the one or more VOCs in the at least one other output to determine the measure of the kidney function of the subject.

In particular, VOCs will be present in blood plasma and will end up in the breath via passive transport via the lung (through liquid/gas exchange). Since the exchange is dependent on the amount of gas (breath) that passes through the lungs, the breathing rate can be used to interpret the measured amount of VOC.

The kidneys will actively filter a certain volume of blood plasma per unit time. Since VOCs are not actively reabsorbed by the kidneys, the amount of VOCs in the urine will be correlated to the volume of blood plasma that is filtered by the kidneys. However, due to active absorption of ions in the water, a fraction of the VOCs will move back into the body. Because this system is driven by osmosis, the measured VOC can also be normalised by measuring the osmolality in urine. The measure of kidney function can be given as follows:

$$\text{Kidney function} = \frac{(VOC_{urine} \times Normalizationfactor)}{(VOC_{breath} \times R)} \qquad (1)$$

where $VOC_{urine}$ is the measurement of the quantity of a VOC in urine, $VOC_{breath}$ is the measurement of the quantity of a VOC in breath and R is the breathing rate.

More generally, any of steps 103, 105 and 107 can comprise comparing measurements of the quantity of VOCs from different outputs to determine the amount of food ingested (step 103), the type of food ingested (step 105) and/or the health status (step 107). In particular, the measurements can be compared by determining a ratio of the measurements of the quantity of the VOCs for a first output (e.g. breath) to the measurements of the quantity of the VOCs for a second output (e.g. urine or sweat).

In some embodiments, any of steps 103, 105 and 107 can comprise comparing the measurements of the quantity of the one or more VOCs over time to previous measurements of the quantity of the one or more VOCs. In some embodiments, the previous measurements may have been obtained during a calibration procedure, for example in which the subject ingested a known quantity and/or certain type of food, and thus a comparison to these previous measurements can indicate the quantity (e.g. based on whether the quantity of VOCs is higher or lower than the previous measurements) and/or whether the certain type of food has been ingested (based on whether the quantity of VOCs is similar to the previous measurements). In some embodiments, the previous measurements are obtained during normal use of the apparatus 50, and thus the comparison can be used to indicate whether a health status is improving, worsening or stable.

In some embodiments, step 101 comprises measuring the quantity of two or more VOCs in the same or different outputs from the subject. For example, the quantity of a first VOC can be measured that is a VOC produced when a food is metabolised or digested by the subject, and a quantity of a second VOC can be measured that is a VOC comprised in the food and that is unchanged by metabolism or digestion in the subject. In this case, step 107 can comprise determining a health status, for example an indication of the liver function, based on changes in the quantity of the first VOC over time relative to changes in the quantity of the second VOC over time.

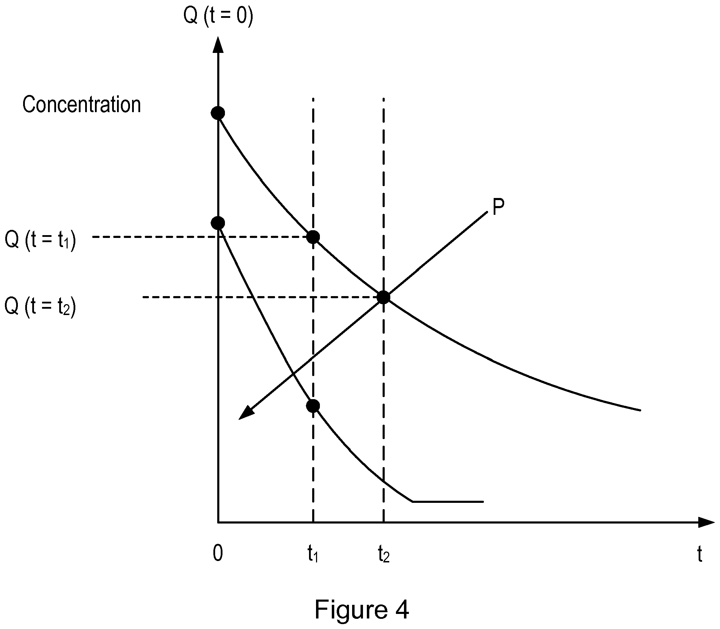

An example of how the quantity (or concentration) Q of a VOC measured in an output of the body can decrease over time is shown in Figure 4. In a simple embodiment, it is assumed that the decay (decrease) curve is known, and follows the following equation:

$$Q(t) = \exp(-t/\tau 1) \qquad (2)$$

where t is time and $\tau 1$ is a time constant.

If the concentration of the VOC, Q, is measured at two different times, $t_1$ and $t_2$: $Q(t_1)$ and $Q(t_2)$, then $\tau$ and Q can be determined for time t = to = 0, which is the time at which the food was ingested, and thus $Q(t_0)$ provides an indication of the quantity and/or type of food ingested by the subject.

It will be appreciated that other means such as curve fitting or regression can be used to determine $Q(t_0)$. For example, when a substance is known to be excreted with two (or more) different rates, e.g. by lungs (in the breath) and skin (in the sweat), more sophisticated relationships could be used, such as multi-exponential fitting:

$$Q(t) = a1 * \exp(-t/\tau 1) + a2 * \exp(-t/\tau 2) + \ldots \qquad (3)$$

where a1 and a2, and τ1 and τ2 are weighing factors and time constants (τ = 1/k, where k is the decay rate) for a first excretion pathway (e.g. via the lungs) and a second excretion pathway (e.g. via the skin) respectively.

Similarly, when a sensor 52 is known to be sensitive to two (or more) VOCs, the measured decay trace might also show a multi-exponential behaviour. In that case, the fitting function then becomes

$$Q(t) = a1 * exp(-t/τ1) + a2 * exp(-t/τ2) + …\qquad\qquad(4)$$

where a1 and a2, and τ1 and τ2 are the weighing factors and time constants for a first VOC of interest and a second VOC of interest respectively. Multi-exponential curve fitting could also be used to correct for a known ventilation (breathing) factor, leading to a more rapid decay rate (decrease) for the measured VOC.

In the case that there is a family of curves parameterised by a parameter (vector) P as shown in Figure 4, the procedure outlined above can be followed and a calibration procedure used to find the required parameters.

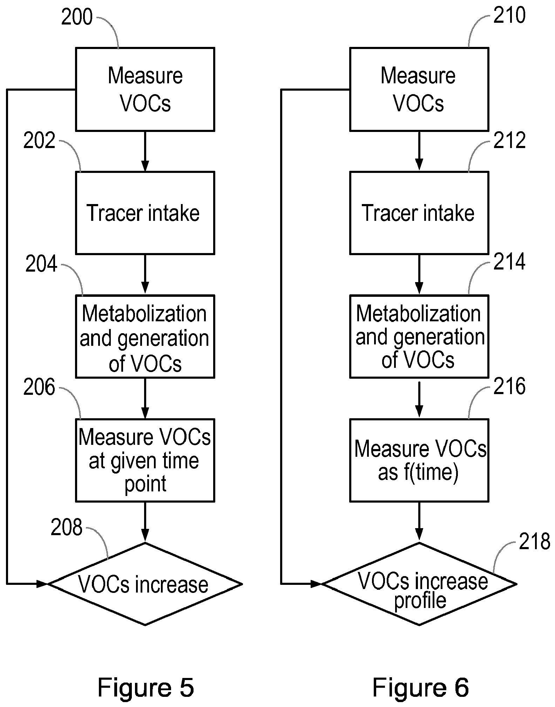

Figures 5, 6 and 7 illustrate several exemplary processes that can be performed according to various embodiments.

The process in Figure 5 can be used to determine if a subject has ingested a particular food. Thus, in this process, an initial measurement of the quantity of VOCs in an output of the subject is measured (block 200) and the food (optionally comprising a tracer element that has been added to the food) is ingested (block 202). The food is then metabolised and VOCs are generated in the body (block 204). The quantity of VOCs in the output of the subject are monitored at a given time point after the food is ingested (block 206), and the measured VOCs compared to the initial VOC measurement in block 200 to determine if the measured quantity of VOCs has increased (block 208). The presence of an increase in the quantity of VOCs will indicate that the subject has ingested food, and the amount of the increase can indicate the amount of food ingested.

As a modification to the process in Figure 5, if malnutrition of the subject is suspected, a certain component of the food can be spiked with a tracer element (e.g. fat, vitamins, vegetables, etc.), and then an increase in the quantity of VOCs will indicate the intake/ingestion of that specific food group/component. The absence of an increase in the quantity of VOCs can indicate that the subject did not eat that component of food (e.g. vegetables).

The process in Figure 6 can be used to determine an indication of the health status of the subject. The metabolic condition of the subject can be useful for assessing the general health of the subject, the impact of chronic diseases and exacerbation events. Thus, in the process of Figure 6, an initial measurement of the quantity of VOCs in an output of the subject is measured (block 210) and the food (optionally comprising a tracer element that has been added to the food) is ingested (block 212). The food is then metabolised and VOCs are generated in the body (block 214). The quantity of VOCs in the output of the subject are monitored over time after the food is ingested (block 216), to determine the VOC quantity as a function of time. In block 218 the VOC profile is compared to the initial VOC measurement in block 210 to determine the change in the quantity of VOCs. The liver is a key organ in the metabolic system and a change of metabolic rate could point to a defect in the liver. Thus, by measuring the decrease of VOC over time the degradation of the VOC components by the subject's liver can be measured. In this case it is preferred to measure a VOC that is at the beginning of the metabolic chain.

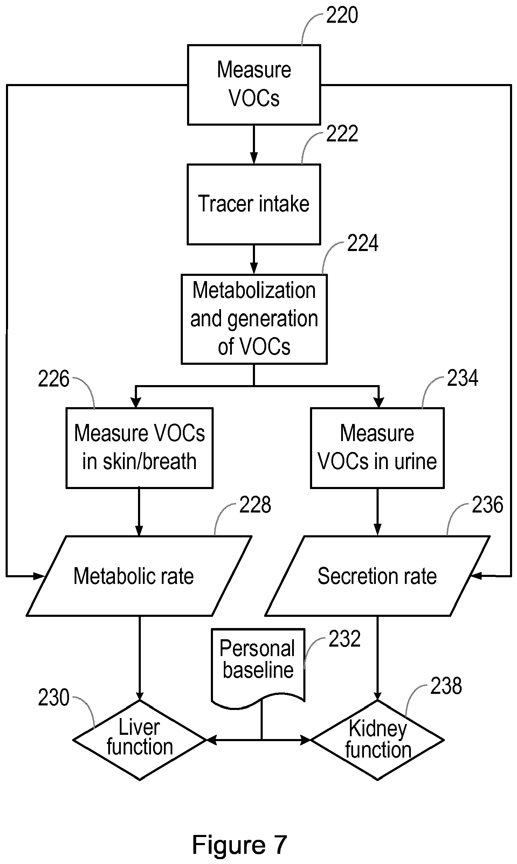

Figure 7 shows a process for estimating the liver function and kidney function of a subject. The VOCs secreted by the kidney can be measured in the urine and the clearance rate of the VOCs from the body via the kidneys will give an indication of the kidney function. When using this in combination with other sources, such as breath and skin, the liver function (i.e. metabolic rate) and the kidney function (i.e. secretion rate) can be separated.

Thus, in the process of Figure 7, an initial measurement of the quantity of VOCs in an output of the subject is measured (block 220) and the food (optionally comprising a tracer element that has been added to the food) is ingested (block 222). The food is then metabolised and VOCs are generated in the body (block 224). The quantity of VOCs in the sweat or breath of the subject are monitored over time after the food is ingested (block 226). In block 228 the metabolic rate is determined by comparing the sweat/breath VOC measurements to the initial VOC measurement in block 220. This comparison provides an indication of the liver function (block 230) and the indication of the liver function can be compared to a personal baseline level for the subject (block 232). In addition the quantity of VOCs in the urine of the subject can be monitored over time after the food is ingested (block 234). In block 236 the secretion rate is determined by comparing the urine VOC measurements to the initial VOC measurement in block 220. This comparison provides an indication of the kidney function (block 238) and the indication of the kidney function can be compared to a personal baseline level for the subject (block 232). As an example, when the level of VOCs in the breath is what is expected (with reference to the personal baseline - block 232) any deviation from the normal values in urine will indicate to reduced clearance and thus potential degradation in kidney function (e.g. kidney failure). Both liver and kidney function are key parameters to investigate in any subject, since they are strongly affected by medication and general health.

In a further example, some diseases can decrease the uptake capacity of the colon. Because the invention can measure the uptake of VOCs by the body, it is possible to measure any loss or reduction in uptake. For example people with Crohn's disease are known to have malnutrition (not enough uptake of certain components and/or micronutrients), and this can be determined from measurements of the quantity of VOCs.

Therefore embodiments of the invention provide a wearable and/or home sensor that monitors the VOCs which are secreted by the body via the lungs, skin, colon, kidneys, etc., preferably in the sweat, breath and/or urine. In some embodiments, a (possibly natural) tracer element can be added to food to be ingested, so that the type and/or amount of food that the subject has eaten can be assessed. The tracer element will be taken up by the body and digested by the metabolic system. The measurements of the amount of VOCs will give a measure of the amount of tracer element that was ingested, and thus the amount of food ingested. Moreover, since the VOCs will only be present when the tracer element is actually taken up by the subject, this enables actual food intake to be measured. The secretion rate from the body of the VOCs from the tracer elements can be used to monitor kidney and liver function.

There is therefore provided an improved method and apparatus for monitoring a subject.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of monitoring a subject, the method comprising:

   obtaining measurements of a parameter relating to one or more volatile organic compounds, VOCs, in one or more outputs of the subject over time, wherein the one or more VOCs are associated with the food or produced while the food is metabolised or digested by the subject; and
   processing the measurements to determine an amount and/or type of food ingested by the subject and/or a health status of the subject.

2. A method as claimed in claim 1, wherein the one or more outputs comprise one or more of breath, urine and sweat.

3. A method as claimed in claim 1 or 2, wherein the health status comprises one or more of a measure of a metabolic rate of the subject, a measure of the kidney function of the subject and a measure of the liver function of the subject.

4. A method as claimed in any of claims 1-3, wherein the step of obtaining comprises obtaining measurements of the parameter relating to the one or more VOCs in urine from the subject, and wherein the step of processing comprises determining a measure of the kidney function of the subject from the measurements of the parameter relating to the one or more VOCs in the urine over time.

5. A method as claimed in claim 4, wherein the step of obtaining comprises obtaining measurements of the parameter relating to the one or more VOCs in at least one other output of the subject over time, and wherein the step of processing comprises comparing the measurements of the parameter relating to the one or more VOCs in the urine over time to the measurements of the parameter relating to the one or more VOCs in the at least one other output to determine the measure of the kidney function of the subject.

6. A method as claimed in any of claims 1-5, wherein the step of obtaining comprises obtaining measurements of the parameter relating to the one or more VOCs in a plurality of outputs of the subject over time, and the step of processing the measurements to determine the health status comprises comparing measurements of the parameter relating to the VOCs for each of the plurality of outputs.

7. A method as claimed in claim 6, wherein the step of comparing comprises determining a ratio of the measurements of the parameter relating to the VOCs for a first output of the plurality of outputs to the measurements of the parameter relating to the VOCs for a second output of the plurality of outputs.

8. A method as claimed in any of claims 1-7, wherein the step of processing the measurements to determine the health status comprises comparing the measurements of the parameter relating to the one or more VOCs over time to previous measurements of the parameter relating to the one or more VOCs in the one or more outputs of the subject over time.

9. A method as claimed in any of claims 1-8, wherein the one or more VOCs comprises a first VOC that is produced when the food is metabolised or digested by the subject, and wherein the step of processing comprises determining a measure of the metabolic rate of the subject and/or the amount and/or type of food ingested by the subject from the measurements of the parameter relating to the first VOC over time.

10. A method as claimed in any of claims 1-9, wherein the one or more VOCs comprises a first VOC that is produced when the food is metabolised or digested by the subject and a second VOC that is comprised in the food and is unchanged by metabolism or digestion in the subject, and wherein the step of processing comprises determining a measure of the liver function of the subject from the measurements of the parameter relating to the first VOC over time relative to the measurements of the parameter relating to the second VOC over time.

11. A method as claimed in any of claims 1-10, wherein the method further comprises the step of:

adding a tracer element to a food to be ingested by the subject, wherein the tracer element is a VOC or produces a VOC when the tracer element is metabolised or digested by the subject.

12. A computer program product comprising a computer readable medium having computer readable code embodied therein, the computer readable code being configured such that, on execution by a suitable computer or processor, the computer or processor is caused to perform the method of any of claims 1-10.

13. An apparatus for monitoring a subject, the apparatus comprising:

one or more sensors for measuring a parameter relating to one or more volatile organic compounds, VOCs, in one or more outputs of the subject over time, wherein the one or more VOCs are associated with the food or produced while the food is metabolised or digested by the subject; and
a processing unit configured to process the measurements to determine an amount and/or type of food ingested by the subject and/or a health status of the subject.

14. An apparatus as claimed in claim 13, wherein the one or more sensors are for measuring the quantity of one or more VOCs in one or more of breath, urine and sweat of the subject.

15. An apparatus as claimed in claim 13 or 14, wherein the health status comprises one or more of a measure of a metabolic rate of the subject, a measure of the kidney function of the subject and a measure of the liver function of the subject.

Figure 1

Figure 2

101

Obtain measurements of a quantity of
one or more VOCs in or more outputs
of the subject over time

103

Process the
measurements to
determine an
amount of food
ingested

105

Process the
measurements to
determine a type
of food ingested

107

Process the
measurements to
determine a health
status of the
subject

Figure 3

Figure 4

EP 3 373 173 A1

Figure 5

Figure 6

Figure 7

Europäisches
Patentamt

European
Patent Office

Office européen
des brevets

## EUROPEAN SEARCH REPORT

Application Number

EP 17 16 0286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | M. SHIRASU ET AL: "The scent of disease: volatile organic compounds of the human body related to disease and disorder", JOURNAL OF BIOCHEMISTRY, vol. 150, no. 3, 19 July 2011 (2011-07-19) , pages 257-266, XP055219359, GB ISSN: 0021-924X, DOI: 10.1093/jb/mvr090 * The whole document, in particular: Pages 257, 258, 261; Tables II and III. * | 1-4,8-15 | INV. G06F19/00 G01N33/48 ADD. A61B5/08 G01N33/497 |
| X | WO 2017/028892 A1 (SIEMENS AG [DE]) 23 February 2017 (2017-02-23) * The whole document, in particular: Pages 1, 3 - 5; Claim 6. * | 1-8, 11-15 | |
| X | US 2016/345910 A1 (AHMAD LUBNA M [US] ET AL) 1 December 2016 (2016-12-01) * Paragraphs [0003], [0004], [0196] - [0200], [0418], [0423], [0424]; Table 1; Figs. 72A, 72B, 73. * | 1-3,8-15 | |

TECHNICAL FIELDS
SEARCHED     (IPC)

G06F
A61B
G01N

The present search report has been drawn up for all claims

| Place of search | Date of completion of the search | Examiner |
|---|---|---|
| Munich | 28 July 2017 | Nagele, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 16 0286

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2017

| Patent document cited in search report | Publication date | Patent family member(s) | Publication date |
|---|---|---|---|
| WO 2017028892 A1 | 23-02-2017 | NONE | |
| US 2016345910 A1 | 01-12-2016 | NONE | |

EPO FORM P0459

For more details about this annex : see Official Journal of the European Patent Office, No. 12/82